# EUROPEAN PATENT SPECIFICATION

(11) **EP 4 085 955 B1**
(45) Date of publication and mention of the grant of the patent: **16.10.2024**
(21) Application number: 19958344.4
(22) Date of filing: 30.12.2019
(51) Int. Cl.: A61M 16/01, A61M 16/10, A61M 16/12

(54) **MEDICAL VENTILATION SYSTEM**
MEDIZINISCHES BELÜFTUNGSSYSTEM
SYSTÈME DE VENTILATION MÉDICALE

(43) Date of publication of application: 09.11.2022
(73) Proprietor: Shenzhen Mindray Bio-Medical Electronics Co., Ltd., Shenzhen, Guangdong 518057 (CN)
(72) Inventor: WU, Xuetao, Shenzhen, Guangdong 518057 (CN); CHEN, Peitao, Shenzhen, Guangdong 518057 (CN)
(74) Representative: KIPA AB
(86) International application number: PCT/CN2019/130215
(87) International publication number: WO 2021/134374

(56) References cited:
- EP-A1- 0 769 304
- WO-A1-2010/130290
- CN-A- 102 580 201
- CN-A- 102 872 517
- CN-A- 105 517 612
- US-A1- 2010 078 018
- US-A1- 2010 078 018
- US-A1- 2015 374 947
- US-B2- 8 459 262

## Description

### TECHNICAL FIELD

The disclosure relates to the technical field of medical devices, and more particularly to a medical ventilation system.

### BACKGROUND

In order to improve oxygenation index of a patient or improve condition of an intubated patient, doctor needs to provide the patient with oxygen ventilation of high flow rate.

### SUMMARY

This disclosure provides a medical ventilation system.

According to an embodiment of this disclosure, a medical ventilation system is provided, which including an input device, a first gas source interface, a second gas source interface, a respiratory line, a drive gas branch, a fresh gas branch, and a ventilation controller; wherein one end of the drive gas branch is connected with the first gas source interface, and the other end of the drive gas branch is connected with the respiratory line;
one end of the fresh gas branch is connected with the first gas source interface and the second gas source interface, and the other end of the fresh gas branch is connected with the respiratory line;
the ventilation controller is configured to control the drive gas branch to provide ventilation support for a patient through the respiratory line, and to control the fresh gas branch to output a fresh gas which contains anesthetic drug to the respiratory line, according to first mode control information which is receive by the input device;
the ventilation controller is further configured to control the drive gas branch to output, a gas which is outputted by the first gas source interface, to the respiratory line; and to control the fresh gas branch to output, at least a gas which is outputted by the first gas source interface or the second gas source interface, to the respiratory line; according to second mode control information which is receive by the input device.

The medical ventilation system provided by the embodiment of the disclosure, in combination with the existing medical ventilation system technology, can not only provide a patient with conventional ventilation support, but also provide the patient with ventilation support of high flow rate, by using the gas line system of the medical ventilation system without adding new devices or configurations. It follows the pipe connection mode of the existing technology without needing to change the existing pipe connection mode, thus reducing the complexity. WO2010/130290 describes a medical ventilation system for the delivery of anesthetic drugs using a fresh gas source and a driving gas source.

It should be understood that the above general description and the following detailed description are only exemplary and explanatory, and do not limit the disclosure.

### BRIEF DESCRIPTION OF THE DRAWINGS

In order to more clearly explain the embodiments of this disclosure or the technical solutions in the prior art, the following briefly introduces the drawings which are needed to be used in the description of the embodiments or the prior art. It is obvious that the drawings in the following description are only some embodiments of this disclosure. For those skilled in the art, other drawings can be obtained from these accompanying drawings without paying any creative works.
FIG. 1 is a structural schematic block diagram of a medical ventilation system provided by an embodiment of this disclosure.
FIG. 2 is a structural schematic diagram of a medical ventilation system provided by an embodiment of this disclosure.
FIG. 3 is a structural schematic diagram of another medical ventilation system provided by an embodiment of this disclosure, which employs a bellows-free technology and is in a first mode.
FIG. 4 is a structural schematic diagram of another medical ventilation system provided by an embodiment of this disclosure, which employs a bellows-free technology and is in a second mode.
FIG. 5 is a structural schematic diagram of a further medical ventilation system provided by an embodiment of this disclosure, which employs a bellows technology and is in a first mode.
FIG. 6 is a structural schematic diagram of a further medical ventilation system provided by an embodiment of this disclosure, which employs a bellows technology and is in a second mode.

### Description of reference numbers:

1000. Medical ventilation system
10. Input device; 11. First gas source interface; 12. Second gas source interface; 13. Respiratory line; 14. Drive gas branch; 15. Fresh gas branch; 16. Ventilation controller; 17. Flow sensor; 18. Expiratory flow sensor; 19. Inspiratory flow sensor;
130. Expiratory branch; 131. Inspiratory branch; 132. Pipeline; 133. Patient; 134. Absorption tank; 1300. Expiratory pipeline; 1301. Expiratory check valve; 1310. Inspiratory pipeline; 1311. Inspiratory check valve;
140. Drive main line; 141. Volumetric exchanger; 142. First flow controller; 143. Expiratory valve; 144. Bellows; 145. Gas storage bypass; 1441. Folding bag;
150. First gas branch; 151.Second gas branch; 152. Mixing branch;
153. Anesthetic evaporator; 154. Evaporator bypass; 155. Second flow controller; 156. Third flow controller.

### DESCRIPTION OF THE PREFERRED EMBODIMENTS

The technical solutions in the embodiments of this disclosure will be described clearly and completely below in combination with the drawings in the embodiments of this disclosure. Obviously, the described embodiments are just some of the embodiments of this disclosure, not all of them. Based on the embodiments in this disclosure, all other embodiments obtained by those skilled in the art without making creative work fall within the protection scope of this disclosure.

In the description of this disclosure, it should be understood that the terms, such as "center", "longitudinal", "transverse", "length", "width", "thickness", "upper", "lower", "front", "rear", "left", "right", "vertical", "horizontal", "top", "bottom", "inner" , "outer", "clockwise", "anticlockwise", which indicate the azimuth or positional relationship, are based on the azimuth or positional relationship shown in the attached drawings. It is only for the convenience of description and simplification the description, rather than indicating or implying that the device or element referred to must have a specific azimuth, be constructed or operated in a specific azimuth. Therefore, it cannot be understood as a limitation of this disclosure. In addition, the terms "first" and "second" are used only for descriptive purposes and cannot be understood as indicating or implying relative importance or implicitly indicating the number of indicated technical features. Thus, the features defined as "first" and "second" may explicitly or implicitly include one or more of the features. In the description of this disclosure, "multiple" means two or more, unless otherwise expressly and specifically defined.

Some embodiments of this disclosure are described in detail below in combination with the accompanying drawings. The following embodiments and features in the embodiments may be combined with each other without conflict.

The most basic and important function of modern medical ventilation system is to assist or control respiration. According to whether the oxygen flow rate provided by the medical ventilation system can fully satisfy the inspiratory requirement of the patient, the medical ventilation systems can be divided into an oxygen supply system with low flow rate and an oxygen supply system with high flow rate.

Specifically, the oxygen supply system with low flow rate has a low oxygen supply rate which cannot satisfies all the oxygen inspiration requirements for the patient, but the patient feels more comfortable and its use is more convenient.

The oxygen supply system with high flow rate has a high oxygen supply rate. During oxygen therapy, it can provide pure oxygen with a maximum flow rate of 60L/min or greater, or a mixed gas with a maximum flow rate of 100L/min, which can fully satisfy all the oxygen inspiration requirements, effectively improve the oxygenation index, and provide the patient with oxygen therapy of high flow rate when necessary.

The embodiment of this disclosure provides a medical ventilation system integrating the conventional function of the medical ventilation system and the high flow rate oxygen supply function. On the premise of adding no new device or configuration, the medical ventilation system is configured to realize the high flow rate oxygen supply scheme at the patient end, and the pipeline connection is simple and convenient for doctor to use.

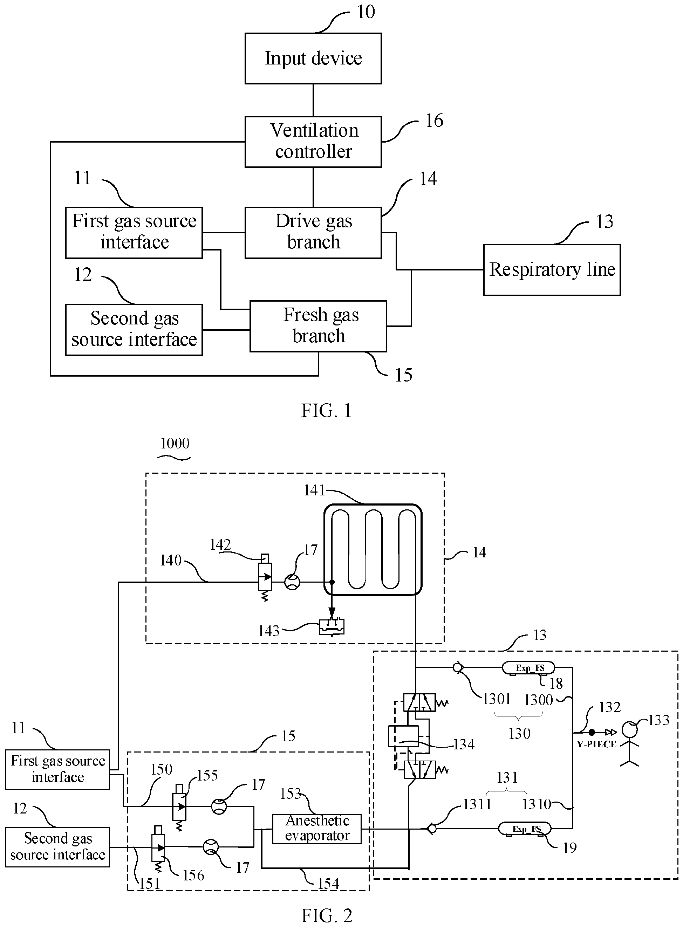

Refer to FIG. 1 and FIG.2. FIG. 1 is a structural schematic block diagram of a medical ventilation system provided by an embodiment of this disclosure. FIG. 2 is a structural schematic diagram of a medical ventilation system provided by an embodiment of this disclosure. The medical ventilation system 1000 includes an input device 10, a first gas source interface 11, a second gas source interface 12, a respiratory line 13, a drive gas branch 14, a fresh gas branch 15, and a ventilation controller 16.

Specifically, the input device 10 may be a switch for switching a first mode and a second mode, and may also be a mechanical knob, an electronic input device (touch screen), or a control panel or other device that receives ventilation control information. It can be understood that the present embodiment is not limited to the above input device 10. The first gas source interface 11 and the second gas source interface 12 can be connected with a gas cylinder or a hospital centralized gas supply system, for receiving oxygen and auxiliary gas. Here, the auxiliary gas may be air or laughing gas, and the likes.

One end of the drive gas branch 14 is connected with the first gas source interface 11, and the other end of the drive gas branch 14 is connected with the respiratory line 13. In the first mode of the medical ventilation system, the drive gas branch 14 provides respiratory support to a patient 133 through the respiratory line 13. When the patient 133 needs to inhale, the drive gas branch 14 outputs gas to the patient 133 through the respiratory line 13. When the patient 133 needs to exhale, the drive gas branch 14 and the respiratory line 13 receive exhaled gas of the patient 133.

One end of the fresh gas branch 15 is connected with the first gas source interface 11 and the second gas source interface 12, and the other end of the fresh gas branch 15 is connected with the respiratory line 13. In the first mode of the medical ventilation system, the fresh gas branch 15 transmits oxygen and auxiliary gas which are outputted from the first gas source interface 11 and the second gas source interface 12 to the anesthetic evaporator 153 for outputting a fresh gas which contains anesthetic drug to the respiratory line 13, and then transmits the fresh gas which contains anesthetic drug to the patient 133 through the respiratory line 13 for realizing the anesthetic drug supplement to the patient 133.

The ventilation controller 16 is configured to control the drive gas branch 14 to provide ventilation support for the patient through the respiratory line 13, and to control the fresh gas branch 15 to output the fresh gas which contains anesthetic drug to the respiratory line 13, according to first mode control information which is receive by the input device 10.

The ventilation controller 16 receives the first mode control information which is outputted by the input device 10 and controls the medical ventilation system 1000 to enter the first mode. In the first mode, the drive gas branch 14 is controlled to provide ventilation support for the patient through the respiratory line 13, and the fresh gas branch 15 is controlled to output the fresh gas which contains anesthetic drug to the respiratory line 13 to realize anesthetization of the patient.

The ventilation controller 16 is further configured to control the drive gas branch 14 to output a first gas to the respiratory line 13, and to control the fresh gas branch 15 to output a second gas to the respiratory line 13, according to second mode control information which is receive by the input device 10.

The ventilation controller 16 receives the second mode control information which is outputted by the input device 10 and controls the medical ventilation system 1000 to enter the second mode. In the second mode, the drive gas branch 14 can output a gas which is outputted by the first gas source interface 11, to the respiratory line 13. Correspondingly, the fresh gas branch 15 can output, at least a gas which is outputted by the first gas source interface 11 or the second gas source interface 12, to the respiratory line 13.

In the second mode, if a gas concentration which is outputted through the respiratory line 13 needs no adjustment, the fresh gas branch 15 can also output the gas which is outputted by the first gas source interface 11 to the respiratory line 13. At this time, the first gas source interface 11 is connected to an oxygen or air source.

In the second mode, if the gas concentration which is outputted through the respiratory line 13 needs to be adjusted, the fresh gas branch 15 can output the gas which is outputted by the second gas source interface 12 to the respiratory line 13, and the fresh gas branch 15 can also output the mixed gases which are outputted by the first gas source interface 11 and the second gas source interface 12 to the respiratory line 13. Since both the drive gas branch 14 and the fresh gas branch 15 can adjust a flow rate of the gas, an oxygen concentration of the oxygen and auxiliary gas which are inputted into the respiratory line 13 can be adjusted adaptively, so as to control the flow rate and/or oxygen supply concentration of the gas which are outputted from the respiratory line 13 to the patient 133.

Specifically, the respiratory line 13 includes an expiratory branch 130 and an inspiratory branch 131. The expiratory branch 130 includes an expiratory pipeline 1300 and an expiratory check valve 1301 arranged at the expiratory pipeline 1300. The expiratory pipeline 1300, the expiratory check valve 1301, and the drive gas branch 14 form an expiratory channel for the exhaled gas of the patient 133. The inspiratory branch 131 includes an inspiratory pipeline 1310, an inspiratory check valve 1311 and an absorption tank 134 arranged at the inspiratory pipeline 1310. In the first mode, when the patient exhales, the expiratory branch 130 and the drive gas branch 14 receive the exhaled gas of the patient. When the patient inhales, the ventilation controller 16 controls the first gas source interface 11 to output gas to provide inspiratory support pressure, filters the exhaled gas in the drive gas branch 14 and the inspiratory branch 131 through the absorption tank 134, mixes it with the fresh gas which is outputted from the fresh gas branch 15, and delivers the mixed gas to the patient 133 to provide inspiratory support for the patient 133. The absorption tank 134 is arranged inside the inspiratory channel for absorbing carbon dioxide in the exhaled gas which is discharged by the drive gas branch 14. In this embodiment, the absorption tank 134 is arranged between the drive gas branch 14 and the inspiratory check valve 1311, and is filled with sodium lime.

It is understandable that in some other embodiments, the absorption tank 134 may be filled with other filter materials for filtering carbon dioxide, which is not limited here. Since there is moisture in the respiratory line 13 during operation, especially the absorption tank 134 releases water in the form of liquid or gas. Therefore, during oxygen supply, the gas is humidified when the oxygen passes through the respiratory line 13, especially the absorption tank 134 which acts as a humidifier.

In the medical ventilation system 1000 designed in this disclosure, when the user selects the first mode through the input device 10, the ventilation controller 16 is configured to control the drive gas branch 14 to provide ventilation support for the patient through the respiratory line 13, and to control the fresh gas branch 15 to output a fresh gas which contains anesthetic drug to the respiratory line 13. When the user selects the second mode through the input device 10, the ventilation controller 16 is configured to control the drive gas branch 14 to output the gas which is outputted by the first gas source interface 11 to the respiratory line 13, and to control the fresh gas branch 15 to output at least the gas which is outputted by the first gas source interface 11 or the second gas source interface 12 to the respiratory line 13. Thus, under the condition of maintaining the conventional anesthetization function of the prior medical ventilation system, when the second mode is selected, the oxygen supply function of a high flow rate can be realized without changing the existing pipeline connection mode, and the complexity of the prior medical ventilation system with the high flow rate can be effectively reduced.

In an optional embodiment, the first mode control information may include one or more of first mode selection information or first mode ventilation control information, the second mode control information includes one or more of second mode selection information or second mode ventilation control information.

Specifically, the first mode selection information is a mode selection information for selecting a conventional function of the medical ventilation system, and the second mode information is a mode selection information for selecting a high flow rate oxygen supply function of the medical ventilation system. Both the first mode selection information and the second mode selection information are inputted by the user through the input device 10.

Accordingly, the first mode ventilation control information may include one or more of conventional ventilation parameters, such as a respiratory rate, an inspiratory flow rate, an inspiratory pressure, an expiratory pressure, an anesthetic drug concentration or a tidal volume, and the user may adjust them as required.

The second mode ventilation control information may include an gas supply flow rate and/or an oxygen supply concentration. Since the second mode is a high flow rate oxygen therapy mode, it is possible to provide high flow rate oxygen supply to the patient 133 by adjusting the air supply flow rate and/or oxygen supply concentration.

In an optional embodiment, the drive gas branch 14 includes a drive main line 140, which includes a first flow controller 142 and an gas storage device which are connected in sequence, and an expiratory valve 143 which is connected to the gas storage device to discharge excess gas in the gas storage device. The gas storage device can adopt either the bellows-free technology or the bellows technology.

Specifically, as shown in FIG. 2, when the bellows-free technology is adopted, the gas storage device can be a volumetric exchanger 141, which is similar to a long and narrow gas duct without rigid separation at both ends and can be interconnected by ventilation. The first flow controller 142 may be a proportional valve for controlling the flow rate of oxygen or auxiliary gas entering the volumetric exchanger 141. When the patient 133 exhales, the expiratory valve 143 discharges the excess gas in the volumetric exchanger 141 to limit the expiratory gas pressure of the patient 133.

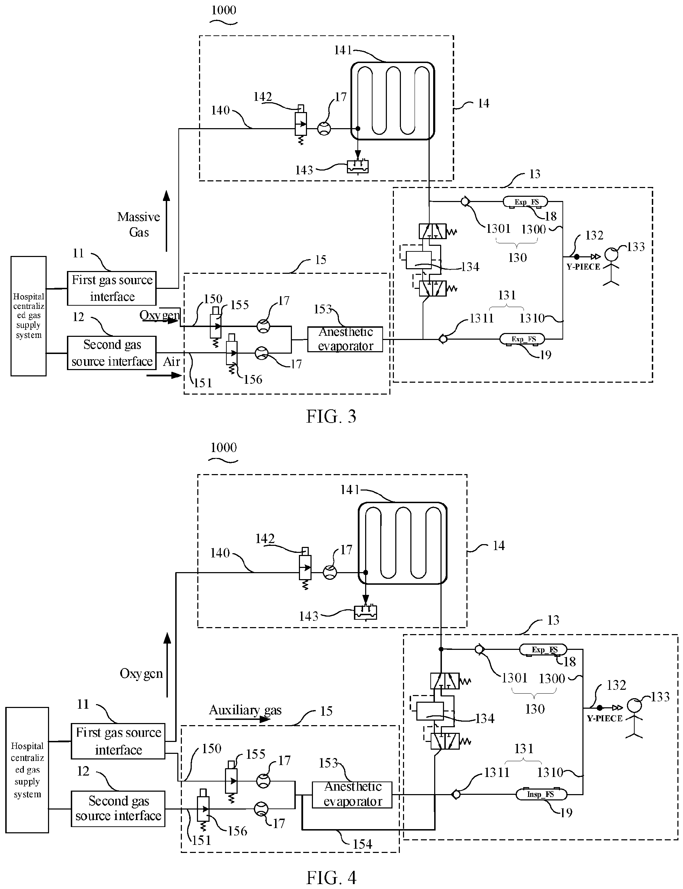

FIG. 3 is a structural diagram of a medical ventilation system in a first mode using a volumetric exchanger 141. As shown in FIG.3, when the ventilation controller 16 receives the first mode control information from the input device 10, it switches the mode of the medical ventilation system to the first mode. The drive gas branch 14 provides respiratory support to the patient 133 through the respiratory line 13. When the patient 133 needs to inhale, the volumetric exchanger 141 outputs a gas to the patient 133 through the respiratory line 13. When the patient 133 needs to exhale, the volumetric exchanger 141 and the respiratory line 13 receive the exhaled gas of the patient 133.

In addition, in the first mode, the fresh gas branch 15 transmits the oxygen and auxiliary gas, which are outputted from the first gas source interface 11 and the second gas source interface 12, through the anesthetic evaporator 153 to output a fresh gas which contains anesthetic drug to the respiratory line 13, and then transmits the fresh gas which contains anesthetic drug to the patient 133 through the respiratory line 13, so as to realize the anesthetic drug supplement to the patient 133. After the patient is anesthetized, the anesthetic evaporator 153 can be turned off. Thus, the medical ventilation system can provide ventilation support and low flow rate oxygen support for patient 133.

FIG. 4 is a structural diagram of a medical ventilation system in a second mode using a volumetric exchanger 141. As shown in FIG.4, when the ventilation controller 16 receives the second mode control information from the input device 10, it switches the mode of the medical ventilation system to the second mode. The drive gas branch 14 outputs the gas which is outputted by the first gas source interface 11 to the respiratory line 13. Accordingly, the fresh gas branch 15 also outputs at least the gas which is outputted by the first gas source interface 11 or the second gas source interface 12 to the respiratory line.

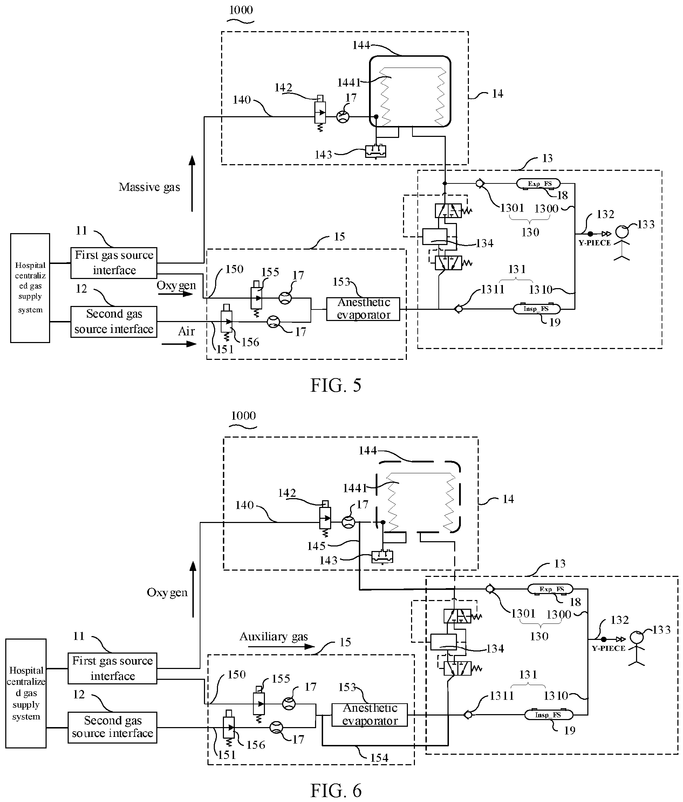

In an optional embodiment, as shown in FIG. 5, which is a structural diagram of the medical ventilation system in the first mode when the bellows technology is adopted. In the first mode, the ventilation controller 16 controls the drive gas branch 14 to provide ventilation support for the patient. Specifically, the bellows 144 provides respiratory support to the patient 133 through the respiratory line 13. When the patient 133 needs to inhale, the bellows 144 outputs gas to the patient 133 through the respiratory line 13. When the patient 133 needs to exhale, the bellows 144 and the respiratory line 13 receive the exhaled gas of the patient 133.

In addition, in the first mode, the fresh gas branch 15 transmits the oxygen and auxiliary gas, which are outputted from the first gas source interface 11 and the second gas source interface 12, through the anesthetic evaporator 153 to output a fresh gas which contains anesthetic drug to the respiratory line 13, and then transmits the fresh gas which contains anesthetic drug to the patient 133 through the respiratory line 13, so as to realize the anesthetic drug supplement and/or provide oxygen support to the patient 133.

FIG.6 is a structural diagram of the medical ventilation system in the second mode when the bellows technology is adopted. As shown in FIG. 6, one end of the bellows 144 is connected with the first gas source interface 11 through the first flow controller 142, and the other end of the bellows 144 is connected with the expiratory branch 130 and the inspiratory branch 131. A folding bag 1441 is arranged inside the bellows 144. The first flow controller 142 controls the drive gas which flows into the bellows 144 through the first gas source interface 11, and the drive gas compresses the folding bag 1441. The expiratory valve 143 is connected with the folding bag 1441 to discharge the excess gas in the folding bag 1441 and control the expiratory pressure of the patient 133.

In the second mode, the gas, which is outputted from the first gas source interface 11, is outputted to the respiratory line 13 through the gas storage bypass 145 of the drive gas branch 14. Accordingly, the fresh gas branch 15 outputs at least the gas which is outputted by the first gas source interface 11 or the second gas source interface 12 to the respiratory line 13.

More specifically, when the ventilation controller 16 receives the second mode control information from the input device 10, it switches the mode of the medical ventilation system to the second mode, closes the main drive line 140 where the bellows 144 is located, opens the gas storage bypass 145, and outputs the gas which is outputted by the first gas source interface 11 to the respiratory line 13 through the gas storage bypass 145. Accordingly, the fresh gas branch 15 outputs at least the gas which is outputted by the first gas source interface 11 or the second gas source interface 12 to the respiratory line 13. The closing of the drive main line 140 where the bellows 144 is located or the opening of the gas storage bypass 145 can be controlled by the corresponding valves on the drive main line 140 or the gas storage bypass 145.

In an optional embodiment, referring to FIGs.2-6, the fresh gas branch 15 includes a first gas branch 150, a second gas branch 151 and a mixing branch 152. One end of the first gas branch 150 is connected with the first gas source interface 11, one end of the second gas branch 151 is connected with the second gas source interface 12, and the other ends of the first gas branch 150 and the second gas branch 151 are connected with the mixing branch 152. The mixing branch 152 is provided with an anesthetic evaporator 153.

Referring to FIG. 3 and FIG. 5, in the first mode, the fresh gas, which is inputted through the first gas branch 150 and the second gas branch 151, is outputted to the respiratory line 13 after passing through the anesthetic evaporator 153. No matter the gas storage device is a bellows 144 or a volumetric exchanger 141, in the first mode, the drive gas branch 14 provides respiratory support to the patient 133 through the respiratory line 13. When the patient 133 needs to inhale, the drive gas branch 14 outputs gas to the patient 133 through the respiratory line 13. When the patient 133 needs to exhale, the drive gas branch 14 and the respiratory line 13 receive the exhaled gas of the patient 133.

In the fresh gas branch 15, one end of the first gas branch 150 is connected with the first gas source interface 11, one end of the second gas branch 151 is connected with the second gas source interface 12, and the other ends of the first gas branch 150 and the second gas branch 151 are connected with the mixing branch 152. In the first mode, after the oxygen and auxiliary gas, which are outputted by the first gas source interface 11 and the second gas source interface 12, are evaporated through the anesthetic evaporator 153, the mixing branch 152 outputs the fresh gas which contains anesthetic drug to the respiratory line 13 and transmitted it to the patient 133 through the respiratory line 13 to realize the supply and supplement of anesthetic drug to the patient 133.

Referring to FIG. 4 and FIG. 6, in the second mode, the drive gas branch 14 can output the gas which is outputted by the first gas source interface 11 to the respiratory line 13. Accordingly, the first gas branch 150 and/or the second gas branch 151 output at least the gas which is outputted by the first gas source interface 11 or the second gas source interface 12 to the respiratory line 13. In one embodiment, the gas which is outputted by the first gas branch 150 and/or the second gas branch 151 can be outputted to the respiratory line 13 through the mixing branch 15. At this time, the anesthetic evaporator 153 is turned off. In another embodiment, the fresh gas branch 15 also includes an evaporator bypass 154, and the gas which is outputted by the first gas branch 150 and/or the second gas branch 151 can also be outputted to the respiratory line 13 through the evaporator bypass 154.

Specifically, the anesthetic evaporator 153 and the mixing branch 152 where the anesthetic evaporator 153 locates, as well as the evaporator bypass 154, can be closed or opened through valve control.

In an optional embodiment, the first gas branch 150 is provided with a second flow controller 155 that controls an input gas flow of the first gas source interface 11, and the second gas branch 151 is provided with a third flow controller 156 that controls an input gas flow of the second gas source interface 12. The second flow controller 155 or the third flow controller 156 may be an electronic flowmeter or a mechanical flowmeter capable of realizing flow control.

It can be understood that the flow sensor 17 can be arranged at the drive gas branch 14, the first gas branch 150 and the second gas branch 151; the expiratory flow sensor 18 can be arranged at the expiratory branch 130 in the respiratory line13; and the inspiratory flow sensor 19 can be arranged at the inspiratory branch 131; to detect the gas flow rates on the corresponding branches.

In the description of the application, it should be noted that, unless otherwise specified and limited, the terms "installation", "connection" and "connected with" should be understood in a broad sense, for example, they can be fixed connections, removable connections, or integrated connections. It can be a mechanical connection or an electrical connection. It can be directly connected or indirectly connected through an intermediate medium. It can be the connection within two components or the interaction relationship between two components. For those skilled in the art, the specific meaning of the above terms in the application can be understood according to the specific circumstances.

In this disclosure, unless otherwise expressly provided and limited, the first feature is "above" or "below" the second feature may include direct contact between the first and second features, or the first and second features may not be in direct contact but through another feature contacting them. Moreover, the first feature is "above", "on" and "at the top of7" the second feature include that the first feature is directly above and obliquely above the second feature, or simply means that the horizontal height of the first feature is higher than the second feature. The first feature is "below", "at the bottom of7" and "under" the second feature, include that the first feature is directly below and obliquely below the second feature, or only indicates that the horizontal height of the first feature is less than that of the second feature.

The above disclosure provides many different embodiments or examples for implementing different structures of this disclosure. In order to simplify the disclosure of this disclosure, components and configurations of specific examples are described above. Of course, they are merely examples and are not intended to limit this disclosure. In addition, this disclosure may repeat reference numerals and/or reference letters in different examples for the purpose of simplification and clarity, and does not in itself indicate the relationship between the various embodiments and/or configurations discussed. In addition, this disclosure provides examples of various specific processes and materials, but those skilled in the art may be aware of the application of other processes and/or the use of other materials.

In the description of this specification, the description referring to the terms "one embodiment", "some embodiments", "schematic embodiments", "examples", "specific examples", or "some examples" means that the specific features, structures, materials, or features described in connection with the embodiments or examples are included in at least one embodiment or example of this application. In this specification, the schematic expression of the above terms does not necessarily refer to the same embodiments or examples. Further, the specific features, structures, materials, or features described may be combined in a suitable manner in any one or more embodiments or examples.

Although the embodiments of this disclosure have been shown and described, those skilled in the art can understand that a variety of changes, modifications, replacements and modifications can be made to these embodiments without departing from the principle and purpose of this disclosure. The scope of configurations is limited by claims in the light of the description.

## Claims

1. A medical ventilation system (1000), comprising:
an input device (10), a first gas source interface (11), a second gas source interface (12), a respiratory line (13), a drive gas branch (14), a fresh gas branch (15), and a ventilation controller (16); wherein one end of the drive gas branch (14) is connected with the first gas source interface (11), and the other end of the drive gas branch (14) is connected with the respiratory line (13);
one end of the fresh gas branch (15) is connected with the first gas source interface (11) and the second gas source interface (12), and the other end of the fresh gas branch (15) is connected with the respiratory line (13);
the ventilation controller (16) is configured to control the drive gas branch (14) to provide ventilation support for a patient through the respiratory line (13), and to control the fresh gas branch (15) to output a fresh gas which contains anesthetic drug to the respiratory line (13), according to first mode control information which is received by the input device (10);
**characterized in that**,
the ventilation controller (16) is further configured to control the drive gas branch (14) to output, a gas which is outputted by the first gas source interface (11), to the respiratory line (13); and to control the fresh gas branch (15) to output, at least a gas which is outputted by the first gas source interface (11) or the second gas source interface (12), to the respiratory line (13); according to second mode control information which is received by the input device (10);
the ventilation controller (16) is further configured to, according to the second mode control information, control the fresh gas branch (15) to output the gas by turning off an anesthetic evaporator (153) or providing an evaporator bypass (154) in the fresh gas branch (15).

2. The medical ventilation system (1000) according to claim 1, **characterized in that**, the first mode control information comprises one or more of first mode selection information or first mode ventilation control information; and
the second mode control information comprises one or more of second mode selection information or second mode ventilation control information.

3. The medical ventilation system (1000) according to claim 2, **characterized in that**,
the first mode ventilation control information comprises one or more of a respiratory rate, an inspiratory flow rate, an inspiratory pressure, an expiratory pressure, an anesthetic drug concentration or a tidal volume; and
the second mode ventilation control information comprises a gas supply flow rate and/or an oxygen supply concentration.

4. The medical ventilation system (1000) according to claim 1, **characterized in that**, the drive gas branch (14) comprises a drive main line (140), wherein the drive main line (140) comprises a first flow controller (142) and a gas storage device which are connected in sequence, and an expiratory valve (143) which is connected to the gas storage device to discharge excess gas in the gas storage device.

5. The medical ventilation system (1000) according to claim 4, **characterized in that**, the drive gas branch (14) further comprises a gas storspage bypass (145), the ventilation controller (16) is configured to control the gas storage bypass (145) to transmit a first gas which is outputted by the first gas source interface (11) to the respiratory line (13), according to the received second mode control information.

6. The medical ventilation system (1000) according to any one of claims 1-5, **characterized in that**, the fresh gas branch (15) comprises a first gas branch (150), a second gas branch (151) and a mixing branch (152); wherein one end of the first gas branch (150) is connected with the first gas source interface (11), one end of the second gas branch (151) is connected with the second gas source interface (12), and the other ends of the first gas branch (150) and the second gas branch (151) are connected with the mixing branch (152);
the mixing branch (152) is provided with the anesthetic evaporator (153);
in a first mode, a fresh gas, which is inputted through the first gas branch (150) and the second gas branch (151), passes through the anesthetic evaporator (153) to form the fresh gas which contains anesthetic drug, and the fresh gas which contains anesthetic drug is outputted to the respiratory line (13);
in a second mode, a gas which is inputted into the mixing branch (152) through the second gas branch (151), is outputted to the respiratory line (13).

7. The medical ventilation system (1000) according to claim 6, **characterized in that**, in the second mode, in order to output the gas, which is inputted into the mixing branch (152) through the second gas branch (151), to the respiratory line (13), the anesthetic evaporator (153) is turned off in the second mode.

8. The medical ventilation system (1000) according to claim 6, **characterized in that**, the fresh gas branch (15) further comprises the evaporator bypass (154), and the ventilation controller (16) is configured to control the gas which is inputted into the mixing branch (152) through the second gas branch (151) to be outputted to the respiratory line (13) through the evaporator bypass (154), according to the received second mode control information.

9. The medical ventilation system (1000) according to any one of claims 6-8, **characterized in that**, the first gas branch (150) is provided with a second flow controller (155), and the second gas branch (151) is provided with a third flow controller (156).

10. The medical ventilation system (1000) according to claim 4, **characterized in that**, the gas storage device is a volumetric exchanger (141), and the first flow controller (142) is a proportional valve.

11. The medical ventilation system (1000) according to claim 4, **characterized in that**, the gas storage device is a bellows (144) arranged inside with a folding bag (1441);
in a first mode, the bellows (144) provides respiratory support to the patient through the respiratory line (13);
in a second mode, one end of the bellows (144) is connected with the first gas source interface (11) through the first flow controller (142), and the other end of the bellows (144) is connected with an expiratory branch (130) and an inspiratory branch (131) of the respiratory line (13);
the first flow controller (142) controls a drive gas which flows into the bellows (144) through the first gas source interface (11), and the drive gas compresses the folding bag (1441); the expiratory valve (143) is connected with the folding bag (1441) to discharge excess gas in the folding bag (1441).

## Patentansprüche

1. Medizinisches Beatmungssystem (1000), mit:
einer Eingabevorrichtung (10), einer ersten Gasquellenschnittstelle (11), einer zweiten Gasquellenschnittstelle (12), einer Atemleitung (13), einem Treibgasschlauch (14), einem Frischgasschlauch (15) und einer Lüftungssteuerung (16); wobei ein Ende des Treibgasschlauchs (14) mit der ersten Gasquellenschnittstelle (11) verbunden ist und das andere Ende des Treibgasschlauchs (14) mit der Atemleitung (13) verbunden ist;
ein Ende des Frischgasschlauchs (15) mit der ersten Gasquellenschnittstelle (11) und der zweiten Gasquellenschnittstelle (12) verbunden ist, und das andere Ende des Frischgasschlauchs (15) mit der Atemleitung (13) verbunden ist;
die Beatmungssteuerung (16) so konfiguriert ist, dass sie den Treibgasschlauch (14) steuert, um eine Beatmungshilfe für einen Patienten durch die Atemleitung (13) bereitzustellen, und den Frischgasschlauch (15) steuert, um ein frisches Gas, das ein Anästhesiemittel enthält, an die Atemleitung (13) abzugeben, gemäß einer ersten Information zur Modussteuerung, die von der Eingabevorrichtung (10) empfangen wird;
**dadurch gekennzeichnet, dass**,
die Beatmungssteuerung (16) ferner so konfiguriert ist, dass sie den Treibgasschlauch (14) so steuert, dass er ein Gas, das von der ersten Gasquellenschnittstelle (11) abgegeben wird, an die Atemleitung (13) abgibt; und dass sie den Frischgasschlauch (15) so steuert, dass er mindestens ein Gas, das von der ersten Gasquellenschnittstelle (11) oder der zweiten Gasquellenschnittstelle (12) abgegeben wird, an die Atemleitung (13) abgibt; gemäß einer zweiten Information zur Modussteuerung, die von der Eingabevorrichtung (10) empfangen wird;
die Beatmungssteuerung (16) ferner so konfiguriert ist, dass sie gemäß der zweiten Information zur Modussteuerung den Frischgasschlauch (15) so steuert, dass er das Gas durch Abschalten eines Narkosemittelverdampfers (153) oder durch Bereitstellen eines Verdampfer-Bypasses (154) in dem Frischgasschlauch (15) abgibt.

2. Medizinisches Beatmungssystem (1000) nach Anspruch 1, **dadurch gekennzeichnet, dass** die Informationen zur Steuerung der Beatmung im ersten Modus eine oder mehrere Informationen zur Auswahl des ersten Modus oder zur Steuerung der Beatmung im ersten Modus beinhalten; und
die Informationen zur Steuerung der Beatmung im zweiten Modus umfasst eine oder mehrere der folgenden Informationen: zweite Betriebsartauswahlinformation oder zweite Betriebsartlüftungssteuerungsinformation.

3. Medizinisches Beatmungssystem (1000) nach Anspruch 2, **dadurch gekennzeichnet, dass**
die Informationen zur Steuerung der Beatmung im ersten Modus eine oder mehrere der folgenden Informationen beinhalten: Atemfrequenz, Einatmungsflussrate, Einatmungsdruck, Ausatmungsdruck, Narkosemittelkonzentration oder Tidalvolumen; und
die Information zur Steuerung der Beatmung im zweiten Modus eine Durchflussrate der Gaszufuhr und/oder eine Sauerstoffzufuhrkonzentration beinhaltet.

4. Medizinisches Beatmungssystem (1000) nach Anspruch 1, **dadurch gekennzeichnet, dass** der Treibgasschlauch (14) eine Antriebs-Hauptleitung (140) aufweist, wobei diese einen ersten Durchflussregler (142) und eine Gasspeichereinrichtung, die hintereinandergeschaltet sind, und ein Ausatmungsventil (143) aufweist, das mit der Gasspeichereinrichtung verbunden ist, um überschüssiges Gas in der Gasspeichereinrichtung abzuleiten.

5. Medizinisches Beatmungssystem (1000) nach Anspruch 4, **dadurch gekennzeichnet, dass** der Treibgasschlauch (14) ferner einen Gasspeicher-Bypass (145) aufweist, wobei die Beatmungssteuerung (16) so konfiguriert ist, dass sie den Gasspeicher-Bypass (145) so steuert, dass er ein erstes Gas, das von der ersten Gasquellenschnittstelle (11) abgegeben wird, an die Atemleitung (13) weiterleitet, und zwar in Übereinstimmung mit der empfangenen Steuerinformation im zweiten Modus.

6. Medizinisches Beatmungssystem (1000) nach einem der Ansprüche 1 bis 5, **dadurch gekennzeichnet, dass** der Frischgasschlauch (15) einen ersten Gasschlauch (150), einen zweiten Gasschlauch (151) und einen Mischschlauch (152) aufweist; wobei ein Ende des ersten Gasschlauchs (150) mit der ersten Gasquellenschnittstelle (11) verbunden ist, ein Ende des zweiten Gasschlauchs (151) mit der zweiten Gasquellenschnittstelle (12) verbunden ist und die anderen Enden des ersten Gasschlauchs (150) und des zweiten Gasschlauchs (151) mit dem Mischschlauch (152) verbunden sind;
der Mischschlauch (152) ist mit einem Narkosemittelverdampfer (153) versehen;
in einem ersten Modus ein Frischgas, das durch den ersten Gasschlauch (150) und den zweiten Gasschlauch (151) zugeführt wird, durch den Narkosemittelverdampfer (153) strömt, um das Frischgas zu bilden, welches ein Narkosemittel enthält, und dieses in die Atemleitung (13) geleitet wird;
in einem zweiten Modus wird ein Gas, das über den zweiten Gasschlauch (151) in den Mischschlauch (152) eingegeben wird, an die Atemleitung (13) ausgegeben.

7. Medizinisches Beatmungssystem (1000) nach Anspruch 6, **dadurch gekennzeichnet, dass** im zweiten Modus der Narkosemittelverdampfer (153) abgeschaltet wird, um das Gas, das über den zweiten Gasschlauch (151) in den Mischschlauch (152) eingegeben wird, an die Atemleitung (13) abzugeben.

8. Medizinisches Beatmungssystem (1000) nach Anspruch 6, **dadurch gekennzeichnet, dass** der Frischgasschlauch (15) ferner den Verdampfer-Bypass (154) umfasst, und dass die Beatmungssteuerung (16) so konfiguriert ist, dass sie das Gas, das über den zweiten Gasschlauch (151) in den Mischschlauch (152) eingegeben wird, um über den Verdampfer-Bypass (154) an die Atemleitung (13) ausgegeben zu werden, entsprechend der empfangenen Steuerinformation des zweiten Modus steuert.

9. Medizinisches Beatmungssystem (1000) nach einem der Ansprüche 6-8, **dadurch gekennzeichnet, dass** der erste Gasschlauch (150) mit einem zweiten Durchflussregler (155) und der zweite Gasschlauch (151) mit einem dritten Durchflussregler (156) versehen ist.

10. Medizinisches Beatmungssystem (1000) nach Anspruch 4, **dadurch gekennzeichnet, dass** die Gasspeichereinrichtung ein Volumentauscher (141) ist und der erste Durchflussregler (142) ein Proportionalventil ist.

11. Medizinisches Beatmungssystem (1000) nach Anspruch 4, **dadurch gekennzeichnet, dass** die Gasspeichervorrichtung ein Faltenbalg (144) mit einem innen angeordneten Faltenbeutel (1441) ist;
der Faltenbalg (144) in einer ersten Betriebsart den Patienten über die Atemleitung (13) bei der Atmung unterstützt;
in einem zweiten Modus ein Ende des Balgs (144) über den ersten Durchflussregler (142) mit der ersten Gasquellenschnittstelle (11) verbunden ist, und das andere Ende des Balgs (144) mit einem Ausatmungsschlauch (130) und einem Einatmungsschlauch (131) der Atemleitung (13) verbunden ist;
der erste Durchflussregler (142) ein Treibgas steuert, welches durch die erste Gasquellenschnittstelle (11) in den Faltenbalg (144) strömt, und das Treibgas den Faltenbeutel (1441) komprimiert; das Ausatmungsventil (143) mit dem Faltenbeutel (1441) verbunden ist, um überschüssiges Gas im Faltenbeutel (1441) abzulassen.

## Revendications

1. Système de ventilation médicale (1000), comprenant :
un dispositif d'entrée (10), une première interface de source de gaz (11), une seconde interface de source de gaz (12), une ligne respiratoire (13), une branche de gaz d'entraînement (14), une branche de gaz frais (15), et une unité de commande de ventilation (16) ; où une première extrémité de la branche de gaz d'entraînement (14) est reliée à la première interface de source de gaz (11), et l'autre extrémité de la branche de gaz d'entraînement (14) est reliée à la ligne respiratoire (13) ;
une première extrémité de la branche de gaz frais (15) est reliée à la première interface de source de gaz (11) et à la seconde interface de source de gaz (12), et l'autre extrémité de la branche de gaz frais (15) est reliée à la ligne respiratoire (13) ;
l'unité de commande de ventilation (16) est configurée pour commander la branche de gaz d'entraînement (14) afin de fournir une assistance de ventilation à un patient par l'intermédiaire de la ligne respiratoire (13), et pour commander la branche de gaz frais (15) afin de délivrer un gaz frais qui contient un médicament anesthésique à la ligne respiratoire (13), selon une information de commande de premier mode laquelle est reçue par le dispositif d'entrée (10) ;
**caractérisé en ce que**,
l'unité de commande de ventilation (16) est en outre configurée pour commander la branche de gaz d'entraînement (14) afin de délivrer un gaz qui est délivré par la première interface de source de gaz (11), à la ligne respiratoire (13) ; et pour commander la branche de gaz frais (15) afin de délivrer au moins un gaz qui est délivré par la première interface de source de gaz (11) ou la seconde interface de source de gaz (12), à la ligne respiratoire (13) ; selon une information de commande de second mode laquelle est reçue par le dispositif d'entrée (10) ;
l'unité de commande de ventilation (16) est en outre configurée pour, selon l'information de commande de second mode, commander la branche de gaz frais (15) afin de délivrer le gaz en éteignant un évaporateur d'anesthésique (153) ou en fournissant une dérivation d'évaporateur (154) dans la branche de gaz frais (15).

2. Système de ventilation médicale (1000) selon la revendication 1, **caractérisé en ce que**, l'information de commande de premier mode comprend une ou plusieurs informations parmi une information de sélection de premier mode et une information de commande de ventilation de premier mode ; et
l'information de commande de second mode comprend une ou plusieurs informations parmi une information de sélection de second mode et une information de commande de ventilation de second mode.

3. Système de ventilation médicale (1000) selon la revendication 2, **caractérisé en ce que**,
l'information de commande de ventilation de premier mode comprend une ou plusieurs informations parmi une fréquence respiratoire, un débit inspiratoire, une pression inspiratoire, une pression expiratoire, une concentration de médicament anesthésique et un volume courant ; et
l'information de commande de ventilation de second mode comprend un débit d'alimentation en gaz et/ou une concentration d'alimentation en oxygène.

4. Système de ventilation médicale (1000) selon la revendication 1, **caractérisé en ce que**, la branche de gaz d'entraînement (14) comprend une ligne principale d'entraînement (140), où la ligne principale d'entraînement (140) comprend une première unité de commande de débit (142) et un dispositif de stockage de gaz qui sont reliés en série, et une valve expiratoire (143) laquelle est reliée au dispositif de stockage de gaz pour évacuer un gaz en excès dans le dispositif de stockage de gaz.

5. Système de ventilation médicale (1000) selon la revendication 4, **caractérisé en ce que**, la branche de gaz d'entraînement (14) comprend en outre une dérivation de stockage de gaz (145), l'unité de commande de ventilation (16) est configurée pour commander la dérivation de stockage de gaz (145) afin de transmettre un premier gaz lequel est délivré par la première interface de source de gaz (11) à la ligne respiratoire (13), selon l'information de commande de second mode reçue.

6. Système de ventilation médicale (1000) selon l'une quelconque des revendications 1 à 5, **caractérisé en ce que**, la branche de gaz frais (15) comprend une première branche de gaz (150), une seconde branche de gaz (151) et une branche de mélange (152) ; où une première extrémité de la première branche de gaz (150) est reliée à la première interface de source de gaz (11), une première extrémité de la seconde branche de gaz (151) est reliée à la seconde interface de source de gaz (12), et les autres extrémités de la première branche de gaz (150) et de la seconde branche de gaz (151) sont reliées à la branche de mélange (152) ;
la branche de mélange (152) est dotée de l'évaporateur d'anesthésique (153) ;
dans un premier mode, un gaz frais, lequel est introduit par la première branche de gaz (150) et la seconde branche de gaz (151), passe à travers l'évaporateur d'anesthésique (153) pour former le gaz frais qui contient un médicament anesthésique, et le gaz frais qui contient le médicament anesthésique est délivré à la ligne respiratoire (13) ;
dans un second mode, un gaz lequel est introduit dans la branche de mélange (152) par le biais de la seconde branche de gaz (151), est délivré à la ligne respiratoire (13).

7. Système de ventilation médicale (1000) selon la revendication 6, **caractérisé en ce que**, dans le second mode, afin de délivrer le gaz, qui est introduit dans la branche de mélange (152) par le biais de la seconde branche de gaz (151), à la ligne respiratoire (13), l'évaporateur d'anesthésique (153) est éteint dans le second mode.

8. Système de ventilation médicale (1000) selon la revendication 6, **caractérisé en ce que**, la branche de gaz frais (15) comprend en outre la dérivation d'évaporateur (154), et l'unité de commande de ventilation (16) est configurée pour commander le gaz qui est introduit dans la branche de mélange (152) par le biais de la seconde branche de gaz (151) à délivrer à la ligne respiratoire (13) par le biais de la dérivation d'évaporateur (154), selon l'information de commande de second mode reçue.

9. Système de ventilation médicale (1000) selon l'une quelconque des revendications 6 à 8, **caractérisé en ce que**, la première branche de gaz (150) est dotée d'une seconde unité de commande de débit (155), et la seconde branche de gaz (151) est dotée d'une troisième unité de commande de débit (156).

10. Système de ventilation médicale (1000) selon la revendication 4, **caractérisé en ce que**, le dispositif de stockage de gaz est un échangeur volumétrique (141), et la première unité de commande de débit (142) est une valve proportionnelle.

11. Système de ventilation médicale (1000) selon la revendication 4, **caractérisé en ce que**, le dispositif de stockage de gaz est un soufflet (144) ayant agencé à l'intérieur un sac repliable (1441) ;
dans un premier mode, le soufflet (144) fournit une assistance respiratoire au patient par le biais de la ligne respiratoire (13) ;
dans un second mode, une première extrémité du soufflet (144) est reliée à la première interface de source de gaz (11) par le biais de la première unité de commande de débit (142), et l'autre extrémité du soufflet (144) est reliée à une branche expiratoire (130) et à une branche inspiratoire (131) de la ligne respiratoire (13) ;
la première unité de commande de débit (142) commande un gaz d'entraînement lequel circule jusqu'au soufflet (144) par le biais de la première interface de source de gaz (11), et le gaz d'entraînement comprime le sac repliable (1441) ; la valve expiratoire (143) est reliée au sac repliable (1441) pour évacuer un gaz en excès dans le sac repliable (1441).
